# FASCICULE DE BREVET EUROPEEN

(11) **EP 0 577 519 B1**
(45) Date de publication et mention de la délivrance du brevet: **10.04.1996**
(21) Numéro de dépôt: 93401724.5
(22) Date de dépôt: 02.07.1993
(51) Int. Cl.: A61K 7/06

(54) **Composition cosmétique à base de maltodextrine pour le maintien et/ou la fixation de la coiffure**
Kosmetische Zusammensetzung enthaltend Maltodextrine zur Erhaltung und/oder zur Fixierung des Frisus
Cosmetic composition containing maltodextrin for dressing and/or for fixing the hair

(30) Priorité: 03.07.1992 FR 9208226
(43) Date de publication de la demande: 05.01.1994
(73) Titulaire: L'OREAL, F-75008 Paris (FR)
(72) Inventeur: Dupuis, Christine, F-75018 Paris (FR); Dubief, Claude, F-78150 Le Chesnay (FR)
(74) Mandataire: Stalla-Bourdillon, Bernard

(56) Documents cités:
- EP-A- 0 074 264
- US-A- 4 971 080
- DATABASE WPIL Week 9116, Derwent Publications Ltd., London, GB; AN 91-110037
- COSMETICS AND PERFUMERY vol. 88, no. 2, Février 1973, pages 31 - 34 NOWAK "Specialty starch products can help solve oily hair problems"

## Description

La présente invention concerne une composition cosmétique pour le maintien et/ou la fixation de la coiffure comprenant au moins un hydrolysat d'amidon dans un milieu cosmétiquement acceptable.

Les compositions de maintien et/ou de fixation de la coiffure renforcent la forme des cheveux dans leur état naturel ou suite à un traitement particulier. Elles se présentent soit sous forme de produits renforçateurs de mises en plis (sur cheveux mouillés), soit sous forme de laques (sur cheveux secs).

Dans les deux cas, on obtient une mise en forme temporaire des cheveux ainsi qu'une protection contre le vent et l'humidité grâce à un film invisible recouvrant la chevelure qui lui confère, par ailleurs, une certaine rigidité et assure donc une meilleure tenue de la coiffure.

La formulation de telles compositions de maintien et/ou de fixation des cheveux est complexe car les exigences sont multiples et souvent contradictoires. Il convient en effet que les critères suivants soient satisfaits :
- bonne brillance
- séchage rapide
- toucher non collant
- non hygroscopicité
- élimination facile au brossage
- absence de cartonnage
- bonne tenue
- application facile.

Les produits utilisés jusqu'à ce jour dans ce domaine n'ont permis de conduire qu'à une satisfaction relative des utilisateurs.

On a maintenant découvert qu'on pouvait éviter ou limiter les inconvénients des produits connus par l'emploi dans une composition de maintien et/ou de fixation de la coiffure d'une classe particulière d'hydrolysats d'amidon.

Les hydrolysats d'amidon sont connus (voir Encyclopedia of Chemical Technology de Kirk-Othmer, 3e Ed., Vol.22, 1978, pp.499 à 521) et sont classés, selon leur apport en dextrose, en d'une part les sirops d'amidon et d'autre part les maltodextrines.

Les sirops d'amidon sont des hydrolysats d'amidon dont le dextrose équivalent (DE) est supérieur à 20 et les maltodextrines sont des hydrolysats d'amidon dont le DE est inférieur à 20.

Le DE est le nombre de grammes de sucres réducteurs (considérés comme du dextrose) pour 100 g de matières sèches du produit. Le DE mesure donc l'intensité de l'hydrolyse de l'amidon puisque, plus le produit contient de petites molécules (telles que dextrose et maltose), plus son DE est élevé. Au contraire, plus le produit contient de grandes molécules (polysaccharides), plus son DE est bas.

Les sirops d'amidon (DE > 20) sont connus dans le domaine des soins capillaires comme étant utiles dans la préparation de compositions pour la fixation des cheveux. On citera à cet égard la demande de brevet DD 284.339 qui décrit, comme agent filmogène, l'association dans un rapport précis d'un sirop d'amidon et d'un tensioactif cationique.

Toutefois, après de nombreuses études, on a constaté que les sirops d'amidon présentaient un pouvoir de tenue insuffisant alors que l'emploi de maltodextrines (DE < 20), permettait d'obtenir des compositions de maintien et/ou de fixation de la coiffure présentant un bien meilleur pouvoir de tenue.

L'invention a donc pour objet une composition cosmétique pour le maintien et/ou la fixation de la coiffure comprenant dans un véhicule cosmétique approprié au moins un hydrolysat d'amidon et au moins un additif cosmétiquement acceptable caractérisée par le fait que l'hydrolysat d'amidon est une maltodextrine ayant un dextrose équivalent (DE) inférieur à 20.

Les compositions selon l'invention permettent de réaliser un maintien et/ou une fixation améliorés des cheveux et donc d'obtenir une bonne tenue de la coiffure grâce à l'emploi des maltodextrines.

Les compositions selon l'invention pour le maintien de la coiffure peuvent être appliquées par exemple comme produit de traitement après une coloration ou une décoloration, après un shampooing, après une permanente ou un défrisage des cheveux.

Elles peuvent également constituer des laques pour le maintien en forme de la chevelure.

Les compositions selon l'invention ont également l'avantage de ne pas provoquer le poissage des cheveux après application.

Selon un mode de réalisation particulier de l'invention, la maltodextrine a un DE inférieur à 14 et de préférence inférieur ou égal à 5.

Les maltodextrines utilisées selon l'invention peuvent être obtenues par l'hydrolyse partielle acide et/ou enzymatique de l'amidon. Divers procédés d'hydrolyse sont connus et ont été décrits de manière générale aux pages 511 et 512 de l'ouvrage Encyclopedia of Chemical Technology de Kirk-Othmer, 3e Ed., Vol.22, 1978.

L'amidon subissant cette hydrolyse peut provenir d'une origine variée mais de préférence du maïs, de fécule de pomme de terre, du tapioca, du riz ou du manioc. Les hydrolysats peuvent subir des modifications chimiques telles que par exemple une acétylation.

Les maltodextrines utilisables selon l'invention se présentent sous forme de poudre blanche ou d'une solution concentrée.

On citera, à titre d'exemple de maltodextrines, les produits vendus par la Société Roquette sous les dénominations de Glucidex® 1W (DE<3), 2 (DE<3), 6 (DE 5-8), 6B (DE 4-8), 9 (DE 8-10), 12 (DE 11-14), 17 (DE 15-18), et sous les dénominations de Glucidex® IT6 (DE 5-8) et IT12 (DE 11-14), l'origine de ces maltodextrines étant l'amidon de maïs.

On peut également citer les produits vendus par la Société National Starch sous les dénominations de N-Zorbit® (DE<5) et Crystal Gum S® (DE<5), l'origine de ces maltodextrines étant l'amidon de manioc.

On peut également citer les produits vendus par la Société Avebe sous les dénominations Paselli SA2® (DE<3) et Amylogum CLS® (DE<3 ), qui proviennent de fécule de pomme de terre.

Selon l'invention, les maltodextrines peuvent être présentes en une proportion comprise entre 0,1 et 20 %, mais de préférence entre 0,5 et 10 % en poids par rapport au poids total de la composition.

La composition selon l'invention peut se présenter sous forme de lotions aqueuses ou hydroalcooliques éventuellement épaissies ou sous forme de gels. Les lotions peuvent être conditionnées sous diverses formes notamment dans des vaporisateurs ou dans des récipients aérosols afin d'assurer une application de la composition sous forme vaporisée ou sous forme de mousse. Une telle forme de conditionnement est indiquée, par exemple, lorsqu'on souhaite obtenir respectivement un spray, une laque ou une mousse pour la fixation des cheveux.

Selon l'invention, le milieu cosmétiquement acceptable est constitué par de l'eau éventuellement en association avec un solvant organique choisi parmi les monoalcools ayant de 1 à 8 atomes de carbone et les polyalcools ou leurs mélanges.

Parmi les monoalcools ayant de 1 à 8 atomes de carbone, on peut citer l'éthanol, l'isopropanol, l'alcool benzylique et l'alcool phényléthylique.

Parmi les polyalcools, on peut mentionner les alkylène glycols tels que l'éthylèneglycol et le propylèneglycol.

Lorsque l'on utilise un solvant, celui-ci est présent dans la composition selon l'invention en une proportion comprise entre 1 et 50 % et de préférence entre 5 et 30 % en poids par rapport au poids total de la composition.

Les additifs cosmétiquement acceptables compatibles avec les maltodextrines sont par exemple des émollients, des lubrifiants, des agents pénétrants, des agents moussants, des agents tensioactifs, des stabilisants, des colorants, des parfums, des agents épaississants, des agents conservateurs, des plastifiants, des agents cationiques, des polymères, des silicones et des protéines animales ou végétales éventuellement quaternisées.

Selon un mode de réalisation particulièrement préférée, la composition selon l'invention contient outre la maltodextrine, une autre substance filmogène tel qu'un polymère filmogène choisi de préférence dans le groupe constitué par :
- le copolymère de vinylpyrrolidone et de chlorure de méthylvinylimidazolium (70/30), vendu en solution aqueuse à 40 % de MA sous la dénomination de Luviquat FC 370® par la Société BASF ;
- le copolymère d'hydroxyéthylcellulose greffé par voie radicalaire par du chlorure de diallyldiméthylammonium, vendu par la Société National Starch sous la dénomination de Celquat L200® ;
- le copolymère vinylméthyléther/anhydride maléïque monoestérifié avec le butanol, vendu à 50 % de matière active (MA) dans l'éthanol sous la dénomination de Gantrez ES 425® par la Société GAF.

Parmi les épaississants utilisables dans les compositions selon l'invention, on peut citer les épaississants naturels tels que les alginates et les pectines, les gommes naturelles telles que la gomme adragante, les gommes de xanthane ou de scléroglucane, les gommes de caroube ou de carraghénanes ou les épaississants synthétiques choisis notamment parmi les polymères d'acide acrylique réticulés ou non et plus particulièrement les acides polyacryliques réticulés par un agent polyfonctionnel tels que le produit vendu sous la dénomination de Carbopol® par la Société Goodrich, les dérivés de cellulose tels que la méthylcellulose, l'hydroxyéthylcellulose, l'hydroxypropylméthylcellulose, les sels de sodium de la carboxyméthylcellulose, ou les copolymères éthylène/anhydride maléïque de haut poids moléculaire.

Selon l'invention, les épaississants peuvent être présents en une proportion comprise entre 0,1 et 10 % et de préférence entre 0,2 et 5 % en poids par rapport au poids total de la composition.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane et leurs mélanges, éventuellement avec au moins un hydrocarbure chloré et/ou fluoré. Parmi ces derniers, on peut citer les composés vendus par la Société Dupont de Nemours sous les dénominations de Fréon® et de Dymel® et en particulier le monofluorotrichlorométhane, le difluorodichlorométhane, le tétrafluorodichloroéthane et les mélanges de ceux-ci.

On peut également utiliser en tant qu'agent propulseur le gaz carbonique, le protoxyde d'azote, l'azote ou l'air comprimé. On utilise de préférence selon l'invention un mélange de n-butane, d'isobutane et de propane éventuellement associé avec du monofluorotrichlorométhane.

Lorsque la composition selon l'invention est conditionnée sous forme d'une laque aérosol, l'agent propulseur représente de préférence de 20 à 80 % du poids total de la composition. Lorsque la composition est conditionnée sous forme d'une mousse aérosol, l'agent propulseur représente de préférence de 5 à 20 % du poids total de la composition.

Une composition particulièrement préférée selon l'invention est constituée par une composition pour fixer les cheveux sous forme d'une mousse ou d'un gel de coiffage.

On va maintenant donner à titre d'illustration et sans aucun caractère limitatif plusieurs exemples de compositions selon l'invention pour le maintien et/ou la fixation de la coiffure.

### EXEMPLE 1 :

On prépare une lotion de mise en forme ayant la composition ci-dessous en procédant au mélange des ingrédients suivants :

| | |
|---|---|
| - Maltodextrine (DE<3) vendue sous la dénomination de Glucidex 2® par la Société Roquette | 2g |
| - Copolymère de vinylpyrrolidone et de chlorure de méthylvinylimidazolium (70/30) vendu en solution aqueuse à 40 % de MA sous la dénomination de Luviquat FC370® par la Société BASF | 0,5 gMA |
| - Silicone aminée vendue en émulsion cationique à 35 % de MA sous la dénomination de Emulsion DC929® par la Société Dow Corning | 0,2 gMA |
| - Ethanol | 10g |
| - Parfum, colorant, conservateur qs | |
| - Eau déminéralisée qsp | 100 g |

Cette lotion s'applique sur les cheveux mouillés après un shampooing et avant la mise en forme. L'application est aisée. Après séchage, la coiffure a une bonne tenue et les cheveux ne collent pas au toucher.

### EXEMPLE 2:

On prépare une mousse de coiffage ayant la composition ci-dessous en procédant au mélange des ingrédients suivants :

| | |
|---|---|
| - Maltodextrine (DE<3) vendue sous la dénomination de Paselli SA 2® par la Société Avebe | 5 g |
| - Copolymère d'hydroxyéthylcellulose greffé par voie radicalaire par du chlorure de diallyl diméthyl ammonium, vendu par la Société National Starch sous la dénomination de Celquat L200® | 0,5 g |
| - Alcool polyvinylique partiellement saponifié (88%) vendu par la Société Hoechst sous la dénomination de Mowiol 40-88® | 0,3 g |
| - Parfum, colorant, conservateur qs | |
| - Eau déminéralisée qsp | 100 g |

### Conditionnement aérosol

90 g de la composition ci-dessus sont conditionnés dans un récipient aérosol en présence de 10 g d'un mélange ternaire de n-butane, isobutane > 55 % et propane vendu sous la dénomination de "Aerogaz 3,2 N®" par la Société Elf-Aquitaine.

Cette mousse s'applique comme dans l'exemple 1 sur cheveux mouillés.

### EXEMPLE 3 :

On prépare un gel de coiffage ayant la composition ci-dessous en procédant au mélange des ingrédients suivants:

| | |
|---|---|
| - Maltodextrine (DE 8-10) vendue sous la dénomination de Glucidex 9® par la Société Roquette | 0,5 g |
| - Copolymère vinylméthyléther/anhydride maléïque monoestérifié avec le butanol, vendu à 50 % de matière active (MA) dans l'éthanol sous la dénomination de Gantrez ES 425® par la Société GAF | 1 gMA |
| - Acide polyacrylique réticulé vendu sous la dénomination de Carbopol 940® (PM 4.000.000) par la Société Goodrich | 1 g |
| - Triéthanolamine qs | pH 7,5 |
| - Parfum, colorant, conservateur qs | |
| - Eau déminéralisée qsp | 100 g |

Ce gel est appliqué sur des cheveux mouillés comme dans l'exemple 1. Après séchage, on observe les mêmes résultats.

### EXEMPLE 4 :

On prépare une laque de coiffage propulsée à l'air comprimé ayant la composition ci-dessous en procédant au mélange des ingrédients suivants :

| | |
|---|---|
| - Maltodextrine acétylée (DE<3) vendue sous la dénomination de Amylogum CLS® par la Société Avebe | 12 g |
| - Parfum, conservateur qs | |
| - Eau déminéralisée qsp | 100 g |

Cette laque est appliquée sur cheveux secs. La coiffure présente une excellente tenue et la laque s'élimine facilement au brossage.

### EXEMPLE 5 :

On prépare une lotion de coiffage conditionnée en flacon pompe ayant la composition suivante :

| | |
|---|---|
| - Maltodextrine (DE 15-18) vendue sous la dénomination de Glucidex 17® par la Société Roquette | 3 gMA |
| - Silicone aminée vendue en émulsion cationique à 35 % de MA sous la dénomination de DC929® par la Société Dow Corning | 0,5 gMA |
| - Ethanol | 24,6 g |
| - Parfum, colorant, conservateur qs | |
| - Eau déminéralisée qsp | 100 g |

Cette lotion est appliquée sur cheveux secs ou mouillés. La coiffure présente une excellente tenue.

## Revendications

1. Composition cosmétique pour le maintien et/ou la fixation de la coiffure comprenant dans un véhicule cosmétique approprié au moins un hydrolysat d'amidon et au moins un additif cosmétiquement acceptable, caractérisée par le fait que l'hydrolysat d'amidon est une maltodextrine ayant un dextrose équivalent (DE) inférieur à 20.

2. Composition selon la revendication 1, caractérisée par le fait que la maltodextrine a un DE inférieur à 14, et de préférence inférieur ou égal à 5.

3. Composition selon l'une quelconque des revendications précédentes, caractérisée par le fait que la maltodextrine est présente en une proportion comprise entre 0,1 et 20 %, et de préférence entre 0,5 et 10 % en poids par rapport au poids total de la composition.

4. Composition selon l'une quelconque des revendications précédentes, caractérisée par le fait qu'elle est sous forme d'une lotion, d'un gel ou conditionnée en aérosol sous forme d'une laque ou d'une mousse.

5. Composition selon l'une quelconque des revendications précédentes, caractérisée par le fait que le milieu cosmétiquement acceptable est choisi parmi l'eau et les mélanges d'eau et d'un solvant organique choisi parmi les monoalcools ayant de 1 à 8 atomes de carbone et les polyalcools et leurs mélanges.

6. Composition selon l'une quelconque des revendications précédentes, caractérisée par le fait que l'additif cosmétiquement acceptable utilisé est choisi parmi les émollients, les lubrifiants, les agents pénétrants, les agents moussants, les agents tensioactifs, les stabilisants, les colorants, les parfums, les agents épaississants, les agents conservateurs, les plastifiants, les agents cationiques, les polymères, les silicones et les protéines animales ou végétales éventuellement quaternisées.

7. Composition selon l'une quelconque des revendications précédentes, caractérisée par le fait qu'elle contient en outre au moins un polymère filmogène.

8. Composition selon l'une quelconque des revendications 1 à 7, caractérisée par le fait qu'elle est conditionnée sous forme d'une laque aérosol, l'agent propulseur représentant de 20 à 80 % du poids total de la composition.

9. Composition selon l'une quelconque des revendications 1 à 7, caractérisée par le fait qu'elle est conditionnée sous forme d'une mousse aérosol, l'agent propulseur représentant de 5 à 20 % du poids total de la composition.

## Claims

1. Cosmetic composition for holding and/or fixing the hairstyle, comprising, in a suitable cosmetic vehicle, at least one starch hydrolysate and at least one cosmetically acceptable additive, characterized in that the starch hydrolysate is a maltodextrin having a dextrose equivalent (DE) of less than 20.

2. Composition according to Claim 1, characterized in that the maltodextrin has a DE of less than 14, and preferably of less than or equal to 5.

3. Composition according to either of the preceding claims, characterized in that the maltodextrin is present in a proportion of between 0.1 and 20%, and preferably of between 0.5 and 10%, by weight relative to the total weight of the composition.

4. Composition according to any one of the preceding claims, characterized in that it is in the form of a lotion or a gel or is packaged as an aerosol in the form of a lacquer or a foam.

5. Composition according to any one of the preceding claims, characterized in that the cosmetically acceptable medium is chosen from water and mixtures of water with an organic solvent chosen from monoalcohols having from 1 to 8 carbon atoms and polyalcohols, and mixtures thereof.

6. Composition according to any one of the preceding claims, characterized in that the cosmetically acceptable additive used is chosen from emollients, lubricants, penetrating agents, foaming agents, surfactants, stabilizers, dyes, fragrances, thickeners, preserving agents, plasticizers, cationic agents, polymers, silicones and animal or plant proteins which are optionally quaternized.

7. Composition according to any one of the preceding claims, characterized in that it also contains at least one film-forming polymer.

8. Composition according to any one of Claims 1 to 7, characterized in that it is packaged in the form of an aerosol lacquer, the propellant representing from 20 to 80% of the total weight of the composition.

9. Composition according to any one of Claims 1 to 7, characterized in that it is packaged in the form of an aerosol foam, the propellant representing from 5 to 20% of the total weight of the composition.

## Patentansprüche

1. Kosmetische Zubereitung zur Beibehaltung und/oder zum Fixieren der Frisur, die in einem kosmetischen geeigneten Träger mindestens ein Stärkehydrolysat und mindestens einen kosmetisch akzeptablen Hilfsstoff enthält, dadurch **gekennzeichnet**, daß das Stärkehydrolysat ein Maltodextrin mit einem Dextroseäquivalent (DÄ) von weniger als 20 darstellt.

2. Zubereitung nach Anspruch 1, dadurch **gekennzeichnet**, daß das Maltodextrin ein DÄ von weniger als 14, vorzugsweise von weniger oder gleich 5 besitzt.

3. Zubereitung nach einem der vorstehenden Ansprüche, dadurch **gekennzeichnet**, daß das Maltodextrin in einem Mengenverhältnis zwischen 0,1 und 20 Gew.-%, vorzugsweise zwischen 0,5 und 10 Gew.-%, in bezug auf das Gesamtgewicht der Zusammensetzung vorhanden ist.

4. Zubereitung nach einem der vorstehenden Ansprüche, dadurch **gekennzeichnet**, daß sie in Form einer Lotion, eines Gels oder als Aerosol in Form eines Lacks oder eines Schaums hergerichtet ist.

5. Zubereitung nach einem der vorstehenden Ansprüche, dadurch **gekennzeichnet**, daß das kosmetisch akzeptable Medium unter Wasser und Mischungen aus Wasser und einem organischen Lösungsmittel ausgewählt ist, das unter einwertigen Alkoholen (Monoalkoholen) mit 1 bis 8 Kohlenstoffatomen und Polyalkoholen sowie deren Gemischen ausgewählt ist.

6. Zubereitung nach einem der vorstehenden Ansprüche, dadurch **gekennzeichnet**, daß der verwendete, kosmetisch akzeptable Hilfsstoff unter Erweichungsmitteln, Gleitmitteln, Penetrationsmitteln, schaumbildenden Stoffen, Tensiden, Stabilisatoren, Farbstoffen, Duftstoffen, Verdickungsmitteln, Konservierungsmitteln, Weichmachern, kationischen Stoffen, Polymeren, Silikonen und tierischen oder pflanzlichen, gegebenenfalls quaternisierten Proteinen ausgewählt ist.

7. Zubereitung nach einem der vorstehenden Ansprüche, dadurch **gekennzeichnet**, daß sie zusätzlich noch mindestens ein filmbildendes Polymer enthält.

8. Zubereitung nach einem der Ansprüche 1 bis 7, dadurch **gekennzeichnet**, daß sie in Form eines Aerosollacks hergerichtet ist, wobei das Treibmittel 20 bis 80 Gew.-% in bezug auf das Gesamtgewicht der Zusammensetzung ausmacht.

9. Zubereitung nach einem der Ansprüche 1 bis 7, dadurch **gekennzeichnet**, daß sie in Form eines Aerosolschaums hergerichtet ist, wobei das Treibmittel 5 bis 20 Gew.-% in bezug auf das Gesamtgewicht der Zusammensetzung ausmacht.
